# EUROPÄISCHE PATENTSCHRIFT

(11) **EP 3 212 096 B1**
(45) Veröffentlichungstag und Bekanntmachung des Hinweises auf die Patenterteilung: **15.07.2020**
(21) Anmeldenummer: 16729762.1
(22) Anmeldetag: 11.05.2016
(51) Int. Cl.: A61B 17/16, A61B 10/02, A61C 8/00

(54) **CHIRURGISCHES HANDGERÄT SOWIE EIN WERKZEUG UND EINE SCHUTZEINRICHTUNG**
SURGICAL HAND-HELD INSTRUMENT, TOOL AND PROTECTION DEVICE
APPAREIL À MAIN CHIRURGICAL, OUTIL ET DISPOSITIF DE PROTECTION

(30) Priorität: 28.05.2015 DE 102015209769
(43) Veröffentlichungstag der Anmeldung: 06.09.2017
(73) Patentinhaber: Zastrow, Frank, 69120 Heidelberg (DE)
(72) Erfinder: Zastrow, Frank, 69120 Heidelberg (DE)
(74) Vertreter: Patent- und Rechtsanwälte Ullrich & Naumann
(86) Internationale Anmeldenummer: PCT/DE2016/200221
(87) Internationale Veröffentlichungsnummer: WO 2016/188522

(56) Entgegenhaltungen:
- EP-A1- 2 543 321
- EP-A1- 2 692 301
- WO-A1-2009/005458
- WO-A2-2012/070822
- US-A1- 2001 009 978

## Beschreibung

Die Erfindung betrifft ein dentalchirurgisches Handgerät mit einem bewegbaren Werkzeug, wobei an einem distalen Ende des Werkzeugs ein Arbeitsbereich ausgebildet ist und wobei zumindest ein distaler Rand des Arbeitsbereichs als Wirkbereich, insbesondere zur spanenden Bearbeitung von Knochen, dient. Des Weiteren betrifft die Erfindung ein dentalchirurgisches Werkzeug mit einem proximalen Ende zur Befestigung an einem chirurgischen Handgerät, wobei an einem distalen Ende des Werkzeugs ein Arbeitsbereich ausgebildet ist und wobei zumindest ein distaler Rand des Arbeitsbereichs als Wirkbereich, insbesondere zur spanenden Bearbeitung von Knochen, dient.

Chirurgisch tätige Zahnärzte, Oralchirurgen und Kieferchirurgen stehen häufig vor der Herausforderung, dass durch Knochenatrophie, durch Unfälle, durch Parodontitis oder durch Zahnextraktion Knochen in der Mundhöhle verloren gegangen ist.

Wenn Zahnimplantate zum Einsatz neuer Zähne geplant werden, so ist es wichtig, dass diese Knochendefizite vorher oder simultan mit der Implantatsetzung aufgebaut werden, damit die Zahnimplantate wieder ein neues Fundament und eine stabile Abstützung im Knochen haben.

Autologer, patienteneigener Knochen gilt dabei nach wie vor als Goldstandard bei knochenaufbauenden Eingriffen. Dies liegt an den Eigenschaften des Knochens, da autologer Knochen sowohl osteogene, osteoinduktive sowie osteokonduktive Eigenschaften miteinander vereint. Das bedeutet, der Knochen hat die Potenz, eigenes Knochengewebe zu bilden, Gefäße zu bilden und wirkt zudem als Leitstruktur für neu gebildeten Knochen. Knochenersatzmaterial hat im Gegensatz zu autologem Knochen keine biologische Potenz und wirkt lediglich osteokonduktiv, das heißt, es wirkt auch als Leitschiene.

Beim Arbeiten mit autologem Knochen sind verschiedene Verfahren vorbekannt. Bei größeren Knochendefiziten bieten sich als zweite intraorale Entnahmestelle grundsätzlich zahnlose Knochenareale an, alternativ der Tuber maxillae, die Spina nasalis anterior, der Gaumen, der Bereich der Kieferhöhlenwand im Oberkiefer oder aber der Unterkiefer, da dieser eher kortikaler Natur ist und die Knochenqualität als sehr gut und stabil gilt. Im Unterkiefer gibt es verschiedene Knochenentnahmestellen, so beispielsweise wiederum zahnlose Areale, das Kinn oder den retromolare Bereich.

Die Knochenentnahme kann hierbei mit verschiedenen Instrumenten erfolgen. Das Konzept der Knochenentnahme ist dabei meist ähnlich.

Entweder werden mit einer sogenannten Lindemannfräse oder mit einem Piezosurgerygerät oder aber mit einer kleinen Säge drei bis vier Sollbruchstellen geschaffen und der Block im Nachhinein mit einem Meisel oder einem anderen Instrument herausgebrochen. Dabei ist nachteilig, dass bei einer Knochenentnahme mehr oder weniger große Gewaltanwendung auf den Kiefer nötig ist. Daher wird dieser Eingriff teilweise vom Arzt gescheut, auch aus dem Grund, da dieses Heraushämmern bzw. -brechen des Knochenblockes aus der jeweiligen Region auch für den Patienten unangenehm ist.

Die EP 2 692 301 A1 zeigt ein medizinisches Werkzeug mit einer in einem Außenschaft drehbar angeordneten Abtrageeinrichtung zum Abtragen von Gewebe. Hierzu weisen der Außenschaft und die Abtrageeinrichtung jeweils eine Schneide auf, die bei einer Rotation der Abtrageeinrichtung gegenüber dem Außenschaft schneidend zusammenwirken.

Des Weiteren ist in der DE 32 02 193 A1 ein chirurgisches Knochenschleifinstrument zur Entnahme von Knochenmaterial und zur Vorbereitung von Knochen zum Einsetzen von Implantaten gezeigt. Das Instrument weist einen als Hohlzylinder ausgebildeten Schleifkopf mit abgerundeter Stirnfläche auf, der an der Außen- und/oder Innenwand mit einem Schleifbelag versehen ist.

Zahnärzte sind aufgrund Ihres Berufes rotierende Instrumente und Bohrer gewohnt. So hat sich auch die sogenannte Trepanfräse etabliert, die an ein Handstück angesteckt wird und einen Kopf aufweist, der als Hohlzylinder ausgebildet ist. An dem distalen Rand des Kopfes sind Zähne zur spanenden Bearbeitung des Knochens ausgebildet. Trepanfräsen werden beispielsweise zur Implantatbettaufbereitung genutzt und weisen einen Durchmesser von ca. 3 mm bis 4 mm auf. Mit Hilfe dieser Fräsen werden äußerst kleine und schmale Bohrzylinder entnommen, die nur bedingt zum Knochenaufbau geeignet sind.

Bei den voranstehend genannten Vorrichtungen müssen zur Knochenentnahme ca. drei bis vier Schnitte bzw. Sollbruchstellen angelegt werden, um das benötigte Knochenstück zu erhalten. Dies bedingt eine starke Belastung für den Patienten und erfordert großes handwerkliches Geschick durch den Operateur. Insbesondere ist dabei zu beachten, dass das umliegende Weichgebewebe, beispielsweise Wange oder Lippe, durch das chirurgische Werkzeug nicht verletzt wird.

Der vorliegenden Erfindung liegt daher die Aufgabe zugrunde, ein chirurgisches Handgerät sowie ein Werkzeug der eingangs genannten Art derart auszugestalten und weiterzubilden, dass mit konstruktiv einfachen Mitteln eine zuverlässige, sichere und für den Patienten schonende Knochenentnahme möglich ist.

Erfindungsgemäß wird die voranstehende Aufgabe durch die Merkmale des Anspruches 1 gelöst. Danach ist das in Rede stehende dentalchirurgische Handgerät dadurch gekennzeichnet, dass der Arbeitsbereich zumindest bereichsweise als Hohlzylindersegment ausgebildet ist, so dass ein Block oder Zylindersegment aus dem Knochen heraustrennbar ist und dass eine Schutzeinrichtung angeordnet ist, um das umliegende Gewebe vor Verletzungen durch das Werkzeug zu schützen.

In erfindungsgemäßer Weise ist zunächst erkannt worden, dass die hier zugrundeliegende Aufgabe durch eine geschickte Ausgestaltung des Arbeitsbereichs in verblüffend einfacher Weise gelöst werden kann. Dabei ist der Arbeitsbereich zumindest bereichsweise als Hohlzylindersegment ausgebildet, um ein etwa halbmondförmiges Knochenstück bzw. Zylindersegment aus dem Knochen heraustrennen zu können. Durch diese Ausgestaltung des Arbeitsbereichs lässt sich das benötigte Knochenstück bzw. Knochensegment aus dem Knochen geradezu "herausschälen". In weiter erfindungsgemäßer Weise ist dabei erkannt worden, dass durch die Anordnung einer Schutzeinrichtung das umliegende Gewebe in idealer Weise vor Verletzungen durch das Werkzeug geschützt ist. Somit ist das chirurgische Handgerät besonders einfach und sicher in der Handhabung. Im Gegensatz zu den aus dem Stand der Technik bekannten Instrumenten bzw. Werkzeugen müssen keine drei bis vier Schnitte bzw. Sollbruchstellen angelegt werden. Es entsteht vielmehr lediglich eine einzige Sollbruchstelle, ein Heraushämmern des Knochens ist nicht notwendig. Der entstehende Block bzw. das Zylindersegment ist sodann ohne größere Gewalteinwirkung herauslösbar bzw. luxierbar, was für den Patienten im Vergleich zu den bekannten Vorrichtungen und Techniken schonender und angenehmer ist. Daher ist die Belastung durch den Eingriff für den Patienten minimal. Weiterhin baut die gesamte Vorrichtung sehr klein, so dass sie sich in idealer Weise für minimalinvasive, insbesondere dentalchirurgische, Eingriffe eignet.

An dieser Stelle sei darauf hingewiesen, dass der Begriff "bewegbar" beispielsweise eine oszillierende longitudinale Schwingung des Werkzeugs oder eine oszillierende (Dreh-)Schwingung um eine in axialer Richtung des Werkzeugs verlaufende Drehachse beschreiben kann. Bei einer oszillierenden Drehschwingung schwingt das Werkzeug bzw. der Arbeitsbereich in einem Winkel α um die Drehachse. Des Weiteren ist denkbar, dass es sich um eine zusammengesetzte Bewegung aus einer oszillierenden longitudinalen Schwingung und einer oszillierenden Drehschwingung handelt.

Weiterhin sei darauf hingewiesen, dass der Begriff "Hohlzylinder" in Bezug auf den Arbeitsbereich im mathematischen Sinne zu verstehen ist. Danach ist ein Zylinder definiert durch eine ebene Kurve C0 in einer Ebene E0, die entlang einer Geraden, die nicht in E0 enthalten ist, um eine feste Strecke verschoben wird. Je zwei sich entsprechende Punkte der Kurven C0 und der verschobenen Kurve C1 werden durch eine Strecke verbunden. Die Gesamtheit dieser parallelen Strecke bildet die zugehörige Zylinderfläche. Bei der Kurve kann es sich bspw. um einen Kreis handeln, sodass ein senkrechter Kreiszylinder entsteht. Des Weiteren ist denkbar, dass die Kurve eine beliebige Form aufweist, bspw. elliptisch oder mehreckig ausgebildet ist. Unter einem "Hohlzylindersegment" ist ein voranstehend definierter Zylinder zu verstehen, der hohl ausgebildet ist und bei dem entlang der - gesamten - Höhe ein Teil der Mantelfläche fehlt.

Im Konkreten kann der gesamte Arbeitsbereich als Hohlzylindersegment ausgebildet sein, wodurch sich die Herstellung und Handhabung erheblich vereinfacht. Dabei ist von besonderem Vorteil, wenn es sich um ein Hohlzylindersegment eines Kreiszylinders handelt, so dass mit einer oszillierenden Drehschwingung des Werkzeugs eine Knochenentnahme möglich ist.

In vorteilhafter Weise weist das Hohlzylindersegment des Arbeitsbereichs, beispielsweise ein Hohlzylindersegment eines Kreiszylinders, eine Segmenthöhe von 2,5 mm bis 3,5 mm, insbesondere 2,7 mm bis 3,3 mm, vorzugsweise 2,9 mm auf. Durch diese Bemaßung des Arbeitsbereichs sowie durch die Schutzeinrichtung ist sichergestellt, dass das Werkzeug nur so weit in den Knochen eindringt, dass eine Verletzung des in dem Knocheninneren verlaufenden Nervs vermieden wird. Dabei kann der Arbeitsbereich des Weiteren derart dimensioniert sein, dass dieser in axialer Richtung maximal 12 mm bis 22 mm, insbesondere 14 mm bis 18 mm, vorzugsweise 15 mm, tief in den Knochen einbringbar ist, um eine Verletzung der innen liegenden Nerven zu verhindern.

Damit das entnommene Knochenstück zur Rekonstruktion eines Implantatlagers geeignet ist, kann der Arbeitsbereich weiterhin derart dimensioniert sein, dass die Kreissehne des Hohlzylindersegments des Arbeitsbereichs eine Länge von 4 mm bis 15 mm, insbesondere von 6 mm bis 12 mm, vorzugsweise von 8 mm, aufweist.

In vorteilhafter Weise ist/sind die Schutzeinrichtung und/oder das Werkzeug lösbar mit dem Handgerät verbunden. Insbesondere können die Schutzeinrichtung und/oder das Werkzeug lösbar mit einem Griff des Handgeräts ausgebildet sein. Bei dem Handgerät bzw. dem Griff kann es sich um ein gängiges Winkelstück oder Handstück handeln, wie es von Zahnärzten bzw. Dentalchirurgen verwendet wird, welches das Werkzeug mit einer entsprechenden Bewegung, bspw. einer Schwingung um eine Drehachse, antreibt. Die Schutzeinrichtung kann an dem Handgerät bzw. dem Handgriff über eine Steckverbindung, eine Schraubverbindung oder eine Bajonettverbindung fixierbar sein. Ferner kann die Schutzeinrichtung in Umfangsrichtung drehbar ausgebildet sein, um eine Anpassung an die Entnahmestelle des Knochens zu ermöglichen. Im Konkreten kann das Werkzeug in das Handgerät bzw. den Handgriff einspannbar sein, wobei zusätzlich das Werkzeug in Umfangsrichtung in unterschiedlichen Stellungen an dem Handgerät bzw. dem Handgriff festlegbar sein kann, um eine Anpassung an die Entnahmestelle des Knochens zu ermöglichen. Alternativ oder zusätzlich kann das Werkzeug, insbesondere dessen Anschlussbereich bzw. Schaft, drehbar mit der Schutzeinrichtung verbunden bzw. gekoppelt sein. Dabei ist der Begriff "drehbar" dahingehend zu verstehen, dass zumindest über einen geringen Winkelbereich hinweg die Schutzeinrichtung und das Werkzeug gegeneinander verdreht werden können. Durch diese Kopplung kann die Schutzeinrichtung zumindest weitestgehend ortsfest angeordnet sein, während das Werkzeug eine oszillierende Bewegung, insbesondere Drehschwingung, ausübt. Zwischen der Schutzeinrichtung und dem Werkzeug kann dazu ein Lager, insbesondere ein Wälzlager, Kugellager, Rillenkugellager bzw. Gleitlager ausgebildet sein. Die Kopplung kann auch über ein elastisches Element, insbesondere einen Ring, vorzugsweise aus Kunststoff bzw. Silikon, erfolgen. Mit dem Verbinden des Anschlussbereiches des Werkzeugs - bspw. einem Schaft - mit dem chirurgischen Handgerät ist auch die Schutzeinrichtung mit dem chirurgischen Handgerät verbunden. Somit dient der Anschlussbereich des Werkzeugs auch als Verbindungselement der Schutzeinrichtung, um nämlich eine vorzugsweise lösbare, Verbindung mit dem chirurgischen Handgerät zu realisieren. Bei einer solchen Ausgestaltung kann die Schutzeinrichtung zusätzlich über ein weiteres Verbindungselement mit dem Handstück bzw. Winkelstück verbunden sein. Ein solches weiteres Verbindungselement hat den zusätzlichen Effekt, dass die Schutzeinrichtung drehfest an dem Handstück bzw. Winkelstück angeordnet ist, so dass ein "Mitdrehen" mit dem Werkzeug vermieden wird.

Des Weiteren ist denkbar, dass die Schutzeinrichtung zumindest bereichsweise als Hohlzylindersegment, beispielsweise eines Kreiszylinders oder eines elliptischen Zylinders, ausgebildet ist. Durch diese konstruktive Maßnahme baut die Schutzeinrichtung extrem klein, sodass sich diese in idealer Weise für dentalchirurgische Anwendungen eignet. Alternativ oder zusätzlich kann die Schutzeinrichtung derart dimensioniert sein, dass sie sich gegenüber der inneren Wandung des Hohlzylindersegments des Werkzeugs in Axialrichtung erstreckt, insbesondere über den distalen Rand hinweg. Durch diese Ausgestaltung ist das umliegende Gewebe vor Verletzungen geschützt ist. Zur weiteren Verbesserung der Handhabbarkeit und zur Vermeidung von Verletzungen des umliegenden Gewebes kann an dem freien Ende der Schutzeinrichtung ein sich radial nach innen erstreckender Vorsprung ausgebildet sein. Dadurch ist der distale Rand des Arbeitsbereichs nochmals besser gegenüber dem umliegenden Gewebe abgeschirmt. Im Konkreten kann der Vorsprung dabei derart ausgebildet sein, dass der distale Rand während seiner Bewegung bzw. Schwingung zeitweise zumindest teilweise radial und axial von der Schutzeinrichtung umgeben ist. Dabei kann der Vorsprung derart ausgebildet sein, dass der distale Rand von einer Art Kragen umschlossen ist. Somit kann ein Teil des Vorsprungs auch radial innerhalb des Arbeitsbereichs verlaufen.

Weiterhin kann an dem freien Ende der Schutzeinrichtung ein radial über das freie Ende hinweg verlaufender, vorzugsweise runder, Steg ausgebildet sein. Durch den Steg ist ein Tiefenschutz realisiert, der ein zu tiefes Eindringen des Werkzeugs in den Knochen verhindert. Alternativ kann die Schutzeinrichtung am freien Ende geschlossen sein. In besonders vorteilhafter Weise kann an der Schutzeinrichtung zumindest eine Austrittsöffnung angeordnet sein, wobei die Austrittsöffnung mit einem in der Schutzeinrichtung verlaufenden Kanal in Strömungsverbindung steht, um das Werkzeug, den Wirkbereich und/oder die Eingriffsstelle mit einem Kühlmedium zu beaufschlagen. Dabei ist denkbar, dass die Austrittsöffnung mit mehreren Kanälen in Strömungsverbindung steht und/oder dass mehrere Austrittsöffnungen vorgesehen sind. Bei dem Kühlmedium kann es sich bspw. um eine NaCI-Lösung handeln. Des Weiteren kann die Austrittsöffnung derart als Düse ausgebildet sein, dass das Kühlmedium weitflächig ausgetragen wird oder nahezu punktuell austritt.

Um dem Operateur eine gute Sicht auf die Eingriffsstelle zu ermöglichen, kann die Schutzeinrichtung in Umfangsrichtung nebeneinander angeordnete Öffnungen aufweisen. Im Konkreten können die Öffnungen oval ausgebildet sein. Alternativ oder zusätzlich kann die Schutzeinrichtung aus einem durchsichtigen Material, vorzugsweise Kunststoff, hergestellt sein. Zur Vereinfachung der Handhabung ist es möglich, an der äußeren Wandung der Schutzeinrichtung Tiefenmarkierungen anzubringen.

In ganz besonders vorteilhafter Weise kann an der Schutzeinrichtung ein sich in axialer Richtung erstreckendes Führungselement, bspw. eine Führungszunge, angeordnet sein. Das Führungselement dient zum leichteren Einführen der Vorrichtung in die "Tasche" zwischen Knochen und Weichgewebe. Das Führungselement kann an dem von dem Handgerät entfernten Ende gebogen ausgebildet sein, um ein besonders leichtes Einbringen in die "Tasche" zu gewährleisten.

In weiter vorteilhafter Weise kann an dem distalen Rand und/oder an der inneren Wandung des Arbeitsbereichs und/oder an der äußeren Wandung des Arbeitsbereichs eine raue Oberfläche ausgebildet sein, die zumindest einen Teil des Wirkbereichs realisiert. Die raue Oberfläche kann mechanisch, elektroerosiv, durch Ätzen oder durch eine Beschichtung mit Diamantkörnern oder Korundkörnern realisiert sein. Im Konkreten kann das Werkzeug bzw. der Arbeitsbereich aus Metall, bspw. Edelstahl, hergestellt sein. Insbesondere Diamantkörner bieten aufgrund ihrer Härte die Möglichkeit einer besonders schonenden und schnellen Bearbeitung des Knochens.

Alternativ oder zusätzlich können an dem distalen Rand Zähne ausgebildet sein, die zumindest einen Teil des Wirkbereichs realisieren. Die Zähne können dabei in Umfangsrichtung an dem distalen Rand vorgesehen sein. Des Weiteren ist denkbar, dass an dem distalen Rand zwei radial versetzt voneinander angeordnete Reihen von Zähnen ausgebildet sind, die bspw. in entgegengesetzte Richtungen geneigt sind, sodass bei einer oszillierenden Drehbewegung des Werkzeugs eine Bearbeitung des Knochens unabhängig von der Drehrichtung möglich ist. Weiterhin kann das Werkzeug jeweils an einem Übergang zwischen distalem Rand und einem der seitlichen Ränder des Hohlzylindersegments abgerundet ausgebildet sein. Somit ist der distale Rand nicht durch "Ecken" begrenzt, die sich bei der Bearbeitung des Knochens mit dem Knochen "verhaken" können.

Zur Vereinfachung der Handhabung des Werkzeugs kann an der äußeren und/oder der inneren Wandung des Arbeitsbereichs eine Markierung zur Visualisierung der Eindringtiefe des Arbeitsbereichs in den Knochen ausgebildet sein. Dies bietet dem Operateur eine einfache Möglichkeit, die Eindringtiefe zu kontrollieren und somit Verletzungen von in dem Knochen liegenden Nerven zu vermeiden. Dabei ist des Weiteren denkbar, dass an dem Arbeitsbereich eine Eindringsperre ausgebildet ist, um ein zu tiefes Eindringen des Werkzeugs in den Knochen zu vermeiden. Diese konstruktive Maßnahme dient ebenfalls dem Schutz der innerhalb des Knochens verlaufenden Nerven. Insbesondere kann die Eindringsperre als an der inneren und/oder äußeren Wandung des Arbeitsbereichs angeordneter Vorsprung realisiert sein. Zur Beaufschlagung des Werkzeugs und/oder der Entnahmestelle mit einem Kühlmedium - bspw. einer NaCI-Lösung - kann an dem Werkzeug mindestens eine Austrittsöffnung ausgebildet sein, wobei die Austrittsöffnung mit einem in dem Werkzeug verlaufenden Kanal in Strömungsverbindung steht.

Eine besonders schonende Bearbeitung des Knochens ist möglich, wenn die Wandung des Arbeitsbereichs eine Dicke von 0,1 mm bis 1,5 mm, insbesondere 0,4 mm bis 0,8 mm aufweist. Als besonders vorteilhaft hat sich eine Dicke von 0,5 mm erwiesen, bei der die Wandung des Arbeitsbereichs die benötigte Stabilität aufweist und dabei eine möglichst dünne Ausgestaltung realisiert ist, um den Knochen schonend zu bearbeiten.

Des Weiteren ist denkbar, dass der distale Rand des Arbeitsbereichs einen konvexen, insbesondere runden oder ovalen, Querschnitt aufweist. Eine solche Geometrie ermöglicht ein besonders "weiches" Eindringen des Werkzeugs in den Knochen. Alternativ kann der distale Rand des Arbeitsbereichs einen eckigen, insbesondere dreieckigen, Querschnitt aufweisen, wodurch auch extrem harte Knochenschichten sehr schonend bearbeitet werden können.

Damit das zu entnehmende Knochenstück die benötigte Größe aufweist, um als Fundament zur Implantatsetzung zu dienen, kann der Arbeitsbereich in axialer Richtung eine Länge von 8 mm bis 23 mm, insbesondere 10 mm bis 18 mm, vorzugsweise 15 mm aufweisen.

Die Wandung des Arbeitsbereichs kann durchgehend massiv ausgebildet sein, sodass im Falle einer rauen Oberfläche an der inneren und/oder äußeren Wandung der Wirkbereich maximiert ist. Ferner kann die Wandung des Arbeitsbereichs Ausnehmungen aufweisen, die bspw. oval ausgebildet sind. Durch die Ausnehmungen wird eine Überhitzung der Entnahmestelle bzw. des Werkzeugs vermieden.

An dem proximalen Ende des Werkzeugs, insbesondere dem Anschlussbereich, kann ein Einlass für eine Kühlflüssigkeit ausgebildet sein, der in Strömungsverbindung mit einem Auslass steht. Der Auslass kann insbesondere im Übergang zwischen proximalem Ende und distalen Ende des Werkzeugs angeordnet sein. Weiterhin ist denkbar, dass über Kühlöffnungen, die sich in Axialrichtung durch den Boden des zylindersegmentförmigen Werkzeugs erstrecken, die Kühlflüssigkeit in das Innere des Werkzeugs einbringbar ist.

In weiter vorteilhafter Weise kann die Schutzeinrichtung zumindest aus zwei Teilen bestehen, wobei die Teile mit einem Klebstoff miteinander verbunden sein können. Um eine Wiederaufbereitung und damit einhergehende Probleme bzgl. der Hygiene zu vermeiden, kann der Klebstoff derart ausgewählt bzw. eingestellt sein, dass er bei einer Wiederaufbereitung bzw. Reinigung, bspw. Autoklavierung, schmilzt, so dass die Schutzeinrichtung nicht mehr verwendbar ist. Alternativ oder zusätzlich kann die Schutzeinrichtung aus einem Material gefertigt sein, dass bei einer Wiederaufbereitung seine Form und/oder Farbe ändert.

Die zugrundeliegende Aufgabe ist des Weiteren durch ein dentalchirurgisches Werkzeug nach Anspruch 11 gelöst. Danach ist das in Rede stehende dentalchirurgische Werkzeug dadurch gekennzeichnet, dass der Arbeitsbereich zumindest bereichsweise als Hohlzylindersegment ausgebildet ist, so dass ein Block oder Zylindersegment aus dem Knochen heraustrennbar ist und dass eine Schutzeinrichtung drehbar mit dem Werkzeug gekoppelt ist, um umliegendes Gewebe vor Verletzungen durch das Werkzeug zu schützen.

Ferner ist eine Schutzeinrichtung für ein an einem chirurgischen Handgerät, insbesondere zur Anwendung in der Dentalchirurgie, angeordnetes Werkzeug angegeben, wobei das Werkzeug an einem distalen Ende einen zumindest bereichsweise als Hohlzylindersegment ausgebildeten Arbeitsbereich aufweist, wobei zumindest ein distaler Rand des Arbeitsbereichs als Wirkbereich, insbesondere zur spanenden Bearbeitung von Knochen, dient, und wobei sich die Schutzeinrichtung in mit dem Handgerät verbundenen Zustand gegenüber der inneren Wandung des Hohlzylindersegments in axialer Richtung erstreckt.

In vorteilhafter Weise kann die Schutzeinrichtung mit dem Werkzeug, insbesondere mit dessen Anschlussbereich bzw. Schaft, drehbar verbunden bzw. gekoppelt sein. Zwischen der Schutzeinrichtung und dem Werkzeug kann dazu ein Lager, insbesondere ein Wälzlager, Kugellager, Rillenkugellager bzw. Gleitlager ausgebildet sein. Die Kopplung kann auch über ein elastisches Element, insbesondere einen Ring, vorzugsweise aus Kunststoff bzw. Silikon, erfolgen. Mit dem Verbinden des Anschlussbereiches des Werkzeugs - bspw. einem Schaft - mit dem chirurgischen Handgerät ist auch die Schutzeinrichtung mit dem chirurgischen Handgerät verbunden. Somit dient der Anschlussbereich des Werkzeugs auch als Verbindungselement der Schutzeinrichtung, um nämlich eine vorzugsweise lösbare, Verbindung mit dem chirurgischen Handgerät zu realisieren. Somit ist auch eine erfindungsgemäße Schutzeinrichtung umfassend ein erfindungsgemäßes Werkzeug angegeben und ausdrücklich Teil dieser Offenbarung. Bei einer solchen Ausgestaltung kann die Schutzeinrichtung zusätzlich über ein weiteres Verbindungselement mit dem Handstück bzw. Winkelstück verbunden sein. Ein solches weiteres Verbindungselement hat den zusätzlichen Effekt, dass die Schutzeinrichtung drehfest an dem Handstück bzw. Winkelstück angeordnet ist, so dass ein "Mitdrehen" mit dem Werkzeug vermieden wird.

Das dentalchirurgische Werkzeug sowie die Schutzeinrichtung kann bzw. können gemäß dem in den Ansprüchen 1 bis 10 beschriebenen Werkzeug bzw. gemäß der in den Ansprüchen 1 bis 10 beschriebenen Schutzeinrichtung ausgestaltet sein und die voranstehend in Bezug auf dieses Werkzeug bzw. diese Schutzeinrichtung genannten Merkmale und Vorteile aufweisen. Des Weiteren kann das Werkzeug gemäß Anspruch 11 sämtliche Merkmale des in der nachfolgenden Figurenbeschreibung beschriebenen Werkzeugs bzw. der Schutzeinrichtung aufweisen.

Es gibt nun verschiedene Möglichkeiten, die Lehre der vorliegenden Erfindung in vorteilhafter Weise auszugestalten und weiterzubilden. Dazu ist einerseits auf die den Ansprüchen 1 und 11 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen, perspektivischen Darstellung ein Ausführungsbeispiel eines erfindungsgemäßen Werkzeugs,
- Fig. 2: in einer schematischen Darstellung ein Ausführungsbeispiel des Querschnitts des distalen Rands eines erfindungsgemäßen Werkzeugs,
- Fig. 3: in einer schematischen Darstellung ein weiteres Ausführungsbeispiel des Querschnitts des distalen Rands eines erfindungsgemäßen Werkzeugs,
- Fig. 4: in einer schematischen, perspektivischen Darstellung ein Ausführungsbeispiel einer Schutzeinrichtung sowie eines Werkzeugs eines erfindungsgemäßen chirurgischen Handgeräts,
- Fig. 5: in einer schematischen, frontalen Darstellung ein weiteres Ausführungsbeispiel einer Schutzeinrichtung sowie eines Werkzeugs eines erfindungsgemäßen chirurgischen Handgeräts,
- Fig. 6: in einer schematischen, geschnittenen Darstellung ein weiteres Ausführungsbeispiel einer Schutzeinrichtung sowie eines Werkzeugs eines erfindungsgemäßen chirurgischen Handgeräts,
- Fig. 7: in einer schematischen, perspektivischen Darstellung ein weiteres Ausführungsbeispiel einer erfindungsgemäßen Schutzeinrichtung,
- Fig. 8: in einer schematischen, perspektivischen Darstellung ein weiteres Ausführungsbeispiel einer erfindungsgemäßen Schutzeinrichtung,
- Fig. 9: in einer schematischen, perspektivischen Darstellung ein weiteres Ausführungsbeispiel einer erfindungsgemäßen Schutzeinrichtung,
- Fig. 10: in einer schematischen Darstellung ein weiteres Ausführungsbeispiel einer erfindungsgemäßen Schutzeinrichtung nebst Werkzeug und
- Fig. 11: in einer schematischen Explosionsdarstellung das Ausführungsbeispiel gemäß Fig. 10.

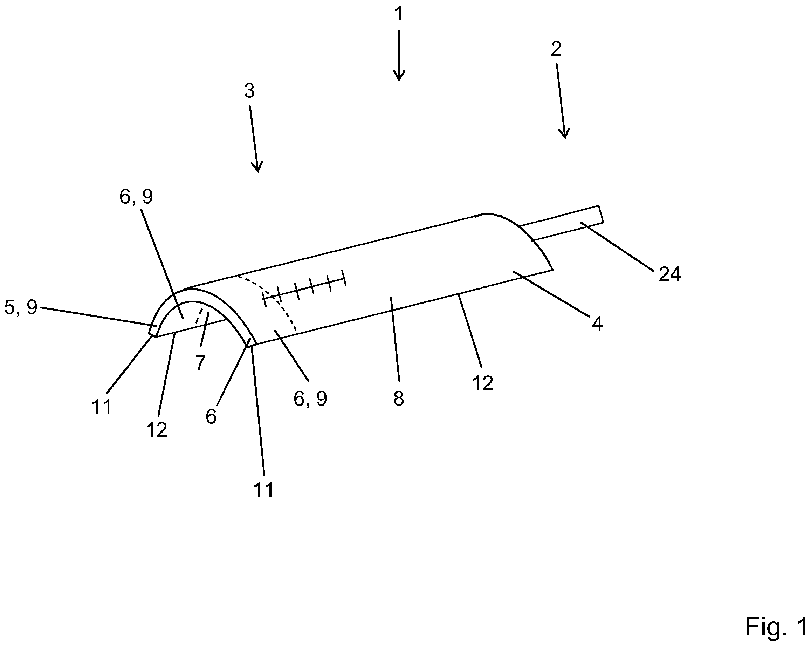

Fig. 1 zeigt in einer schematischen, perspektivischen Darstellung ein Ausführungsbeispiel eines erfindungsgemäßen Werkzeugs 1. Das chirurgische Werkzeug 1 weist ein proximales Ende 2 zur Befestigung an einem chirurgischen Handgerät bzw. Handgriff auf. An dem distalen Ende 3 des Werkzeugs 1 ist ein Arbeitsbereich 4 ausgebildet. Der distale Rand 5 des Arbeitsbereichs 4 dient als Wirkbereich 9, insbesondere zur spanenden Bearbeitung von Knochen. Aus Fig. 1 geht dabei eindeutig hervor, dass der Arbeitsbereich 4 als Hohlzylindersegment ausgebildet ist. Im hier dargestellten Ausführungsbeispiel handelt es sich um ein Hohlzylindersegment eines Kreiszylinders. Dabei ist jedoch denkbar, dass es sich um einen beliebig geformten Zylinder handelt, bspw. auch einen elliptischen Zylinder.

An dem distalen Rand 5 ist eine raue Oberfläche 6 vorgesehen. Im hier dargestellten Ausführungsbeispiel ist an der inneren Wandung 7 und der äußeren Wandung 8 ebenfalls eine raue Oberfläche 6 realisiert. Die raue Oberfläche 6 kann bspw. durch eine Beschichtung mit Diamantkörnern oder Korundkörnern hergestellt sein. Die Ausdehnung der rauen Oberfläche 6 auf der inneren Wandung 7 und der äußeren Wandung 8 ist jeweils durch eine gestrichelte Linie dargestellt. Jedoch ist denkbar, dass die gesamte innere Wandung 7 und/oder äußere Wandung 8 mit einer rauen Oberfläche 6 ausgebildet ist. Der Wirkbereich 9 ist insgesamt durch die raue Oberfläche 6 an dem distalen Rand 5, der inneren Wandung 7 und der äußeren Wandung 8 gebildet. An dieser Stelle sei ausdrücklich darauf hingewiesen, dass der distale Rand 5 an Stelle einer rauen Oberfläche Zähne aufweisen kann, die zumindest einen Teil des Wirkbereichs 9 realisieren.

Im Ausführungsbeispiel gemäß Fig. 1 ist der distale Rand 5 flach ausgebildet, d. h. weist einen flachen bzw. graden Querschnitt auf.

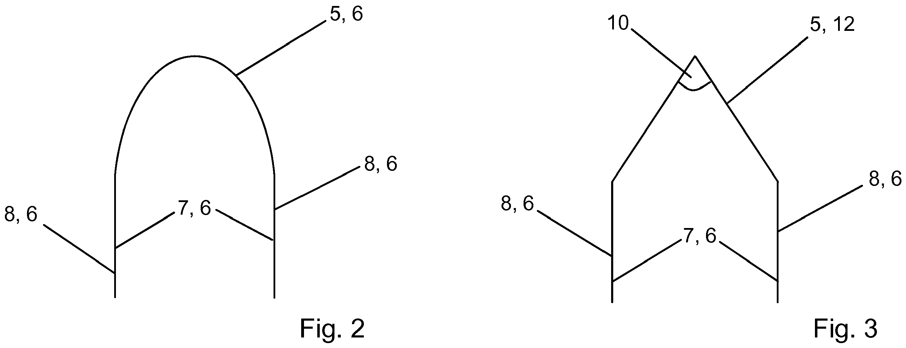

Fig. 2 zeigt in einer schematischen Darstellung ein weiteres Ausführungsbeispiel des Querschnitts des distalen Rands 5 eines Werkzeugs 1. Der distale Rand 5 hat gemäß Fig. 2 einen runden, nämliche ovalen Querschnitt. Diese Konstruktion zeichnet sich durch ein besonders leichtes Eindringen des distalen Rands 5 in den Knochen aus. Des Weiteren sind in Fig. 2 sowohl die innere Wandung 7 als auch die äußere Wandung 8 und der distale Rand 5 mit einer rauen Oberfläche 6 versehen. Jedoch kann lediglich der distale Rand 5 oder der distale Rand 5 und die innere Wandung 7 oder der distale Rand 5 und die äußere Wandung 8 mit einer rauen Oberfläche 6 ausgebildet sein. Des Weiteren können an dem distalen Rand 5 Zähne ausgebildet sein.

Fig. 3 zeigt in einer schematischen Darstellung ein weiteres Ausführungsbeispiel des Querschnitts des distalen Rands 5 eines Werkzeugs 1. Im Gegensatz zu den in den Fig. 1 und 2 dargestellten Ausführungsbeispielen ist der Querschnitt des distalen Rands 5 in Fig. 3 eckig ausgebildet, weist nämlich einen Winkel 10 auf. An dieser Stelle sei darauf hingewiesen, dass der Übergang zwischen dem distalen Rand 5 und der inneren Wandung 7 bzw. der äußeren Wandung 8 wie in Fig. 3 dargestellt eckig ausgestaltet sein kann. Des Weiteren kann dieser Übergang auch abgerundet realisiert sein. Weiterhin sind in Fig. 3 sowohl die innere Wandung 7 als auch die äußere Wandung 8 und der distale Rand 5 mit einer rauen Oberfläche 6 versehen. Jedoch kann lediglich der distale Rand 5 oder der distale Rand 5 und die innere Wandung 7 oder der distale Rand 5 und die äußere Wandung 8 mit einer rauen Oberfläche 6 ausgebildet sein. Weiterhin können an dem distalen Rand 5 Zähne ausgebildet sein, insbesondere zur spanenden Bearbeitung von Knochen.

In Bezug auf die Fig. 1 bis 6 sei ausdrücklich darauf hingewiesen, dass das Werkzeug 1 jeweils an den Übergängen 11 zwischen dem distalen Rand 5 und einem der seitlichen Ränder 12 des Hohlzylindersegments abgerundet ausgebildet sein kann.

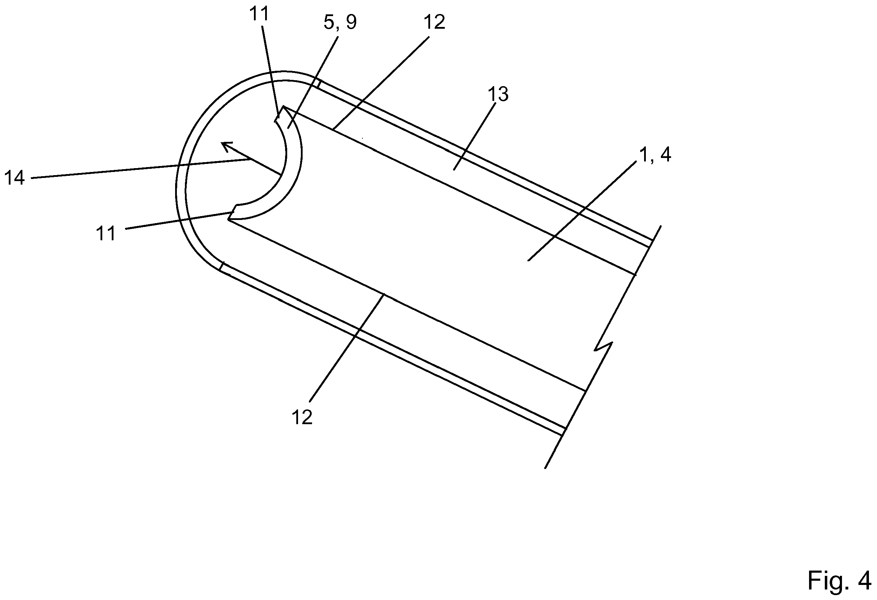

Fig. 4 zeigt in einer schematischen, perspektivischen Darstellung ein Ausführungsbeispiel einer Schutzeinrichtung 13 sowie eines Werkzeugs 1 eines erfindungsgemäßen chirurgischen Handgeräts. Zur Vereinfachung der Darstellung ist in Fig. 4 sowie den nachfolgenden Figuren auf die Darstellung des Handgeräts, d. h. des Winkelstücks bzw. Handstücks, an dem die Schutzeinrichtung 13 und das Werkzeug 1 festgelegt sind, nicht gezeigt. Aus Fig. 1 ist ersichtlich, dass das Werkzeug 1 als Hohlzylindersegment ausgebildet ist. Das Werkzeug 1 kann dabei den in den Fig. 1 bis 3 gezeigten Werkzeugen 1 entsprechen, so dass unter Bezugnahme auf die voranstehende Figurenbeschreibung sich weitere Ausführungen erübrigen. Die Schutzeinrichtung 13 ist ebenfalls als Hohlzylindersegment ausgebildet. Im hier dargestellten Ausführungsbeispiel ist die Schutzeinrichtung 13 als Hohlzylindersegment eines Kreiszylinders dargestellt.

Jedoch kann es sich auch um ein Hohlzylindersegment eines beliebigen Zylinders, bspw. eines elliptischen Zylinders handeln.

Die Schutzeinrichtung 13 erstreckt sich gegenüber der inneren Wandung 7 des Arbeitsbereichs 4, d.h. des Hohlzylindersegments, in Axialrichtung, so dass das umliegende Gewebe vor Verletzung durch das Werkzeug 1 geschützt ist. Des Weiteren ist in Fig. 4 eine Drehachse 14 gezeigt, um die das Werkzeug 1 schwingt. Ferner kann das Werkzeug 1 alternativ oder zusätzlich eine longitudinale Schwingung parallel zur Drehachse 14 ausführen.

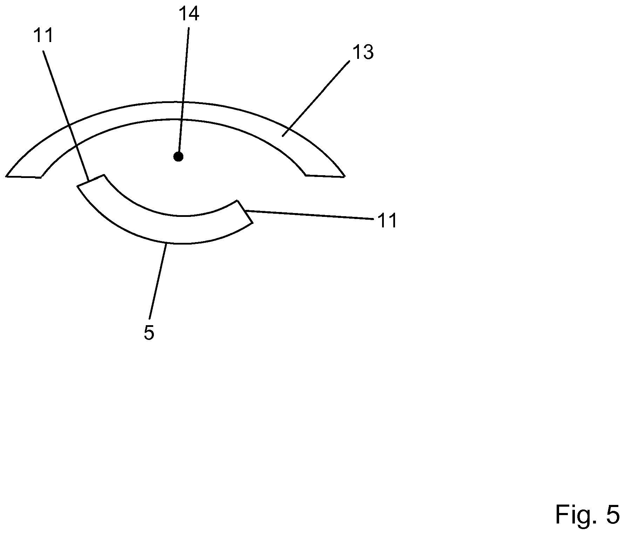

Fig. 5 zeigt in einer schematischen, frontalen Darstellung ein weiteres Ausführungsbeispiel einer Schutzeinrichtung 13 sowie eines Werkzeugs 1 eines erfindungsgemäßen chirurgischen Handgeräts. Dabei ist nochmals deutlich zu erkennen, dass die Schutzeinrichtung 13 den Arbeitsbereich 4 derart umgibt, dass das umliegende Gewebe vor Verletzungen geschützt ist.

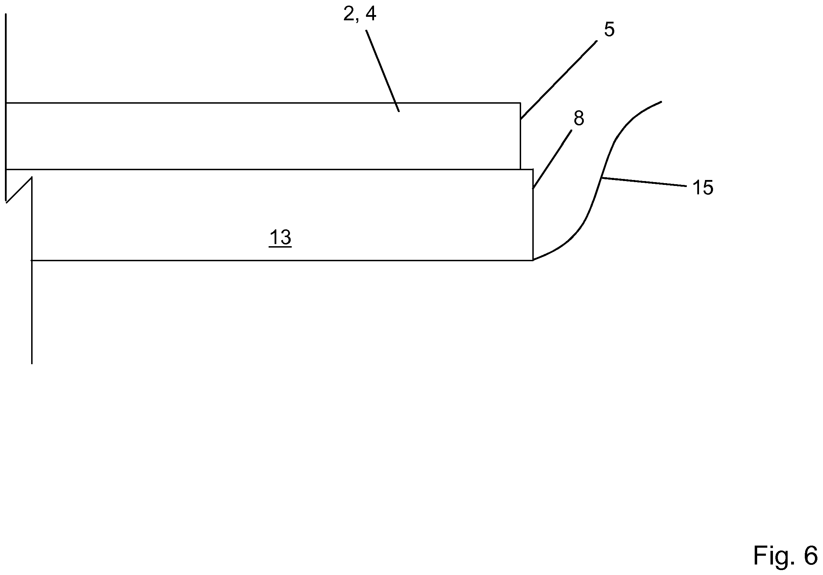

Fig. 6 zeigt in einer schematischen, geschnittenen Darstellung ein weiteres Ausführungsbeispiel einer Schutzeinrichtung 13 sowie eines Werkzeugs 1 eines erfindungsgemäßen chirurgischen Handgeräts. Das Ausführungsbeispiel gemäß Fig. 6 entspricht den Ausführungsbeispielen gemäß den Fig. 4 und 5, wobei zusätzlich ein sich in Axialrichtung erstreckendes Führungselement 15 angeordnet ist. Das Führungselement 15 dient zur leichteren Einführung in die Tasche zwischen Weichgewebe und Knochen. Dabei kann das Führungselement 15 aus dem gleichen Material wie die Schutzeinrichtung 13 hergestellt und insbesondere als integraler Bestandteil der Schutzeinrichtung 13 ausgebildet sein.

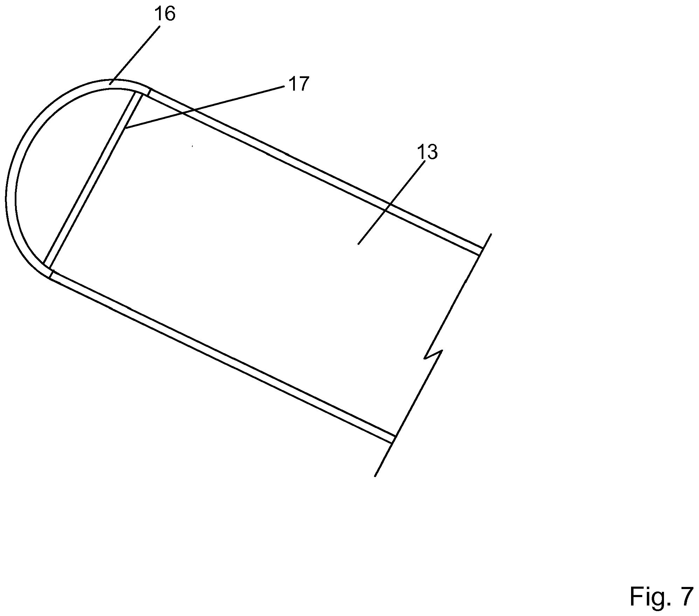

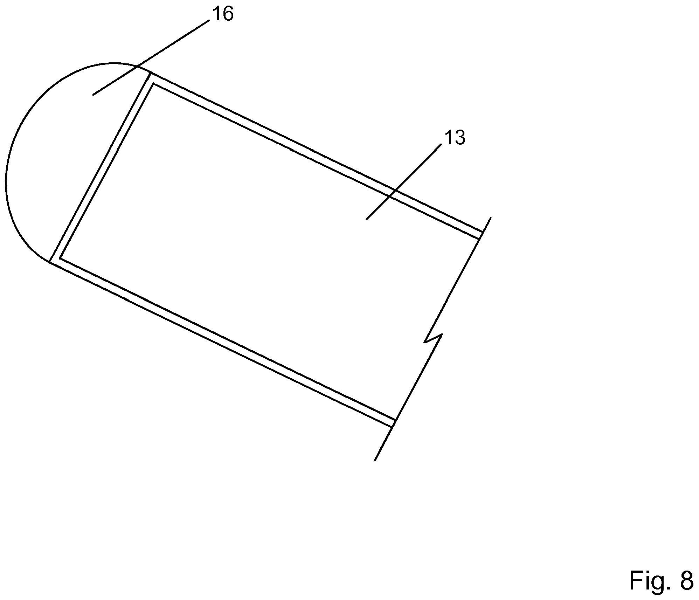

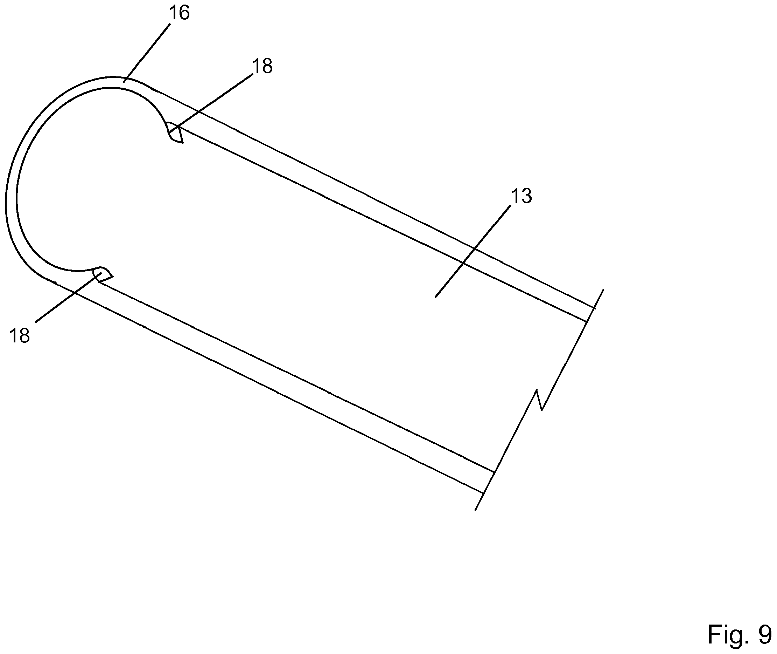

Fig. 7 zeigt in einer schematischen, perspektivischen Darstellung ein weiteres Ausführungsbeispiel einer erfindungsgemäßen Schutzeinrichtung 13. Zur Vereinfachung der Darstellung ist in Fig.7 sowie den nachfolgenden Fig. 8 und 9 das Werkzeug 1 nicht dargestellt. Im Unterschied zu der in den voranstehenden Figuren gezeigten Schutzeinrichtung 13 ist an dem freien Ende 16 der Schutzeinrichtung 13 ein radial über das freie Ende 16 hinweg verlaufender Steg 17 ausgebildet. Der Steg 17 dient dazu, ein zu tiefes Eindringen des Werkzeugs 1 in den Knochen zu verhindern.

Fig. 8 zeigt in einer schematischen, perspektivischen Darstellung ein weiteres Ausführungsbeispiel einer erfindungsgemäßen Schutzeinrichtung 13. Die Schutzeinrichtung 13 entspricht der Schutzeinrichtung 13 gemäß Fig. 7, wobei an Stelle eines Stegs 17 das freie Ende 16 der Schutzeinrichtung 13 komplett verschlossen ist.

Fig. 9 zeigt in einer schematischen, perspektivischen Darstellung ein weiteres Ausführungsbeispiel einer erfindungsgemäßen Schutzeinrichtung 13. An dem freien Ende 16 der Schutzeinrichtung 13 ist ein sich radial nach innen erstreckender Vorsprung 18 ausgebildet. Der Vorsprung 18 hat in diesem Ausführungsbeispiel die Form eines Kragens, erstreckt sich nämlich nicht nur radial nach innen sondern auch axial in Richtung des Werkzeugs 1, so dass der distale Rand 5 des Arbeitsbereichs 4 zumindest in einigen Positionen während der Bewegung des Werkzeugs von dem Vorsprung 18 umschlossen ist. Dabei sei ausdrücklich darauf hingewiesen, dass der Vorsprung 18 auch derart ausgebildet sein kann, dass er sich lediglich radial nach innen erstreckt und somit den distalen Rand 5 des Werkzeugs 1 in Axialrichtung abdeckt. Des Weiteren sei angemerkt, dass die Schutzeinrichtung 13 gemäß Fig. 9 ein größeres Hohlzylindersegment darstellt als die in den Fig. 4 bis 8 gezeigten Schutzeinrichtungen. Das Hohlzylindersegment kann jedoch eine beliebige Größe aufweisen, d.h. sich über einen kleineren oder größeren Bereich in Umfangsrichtung erstrecken.

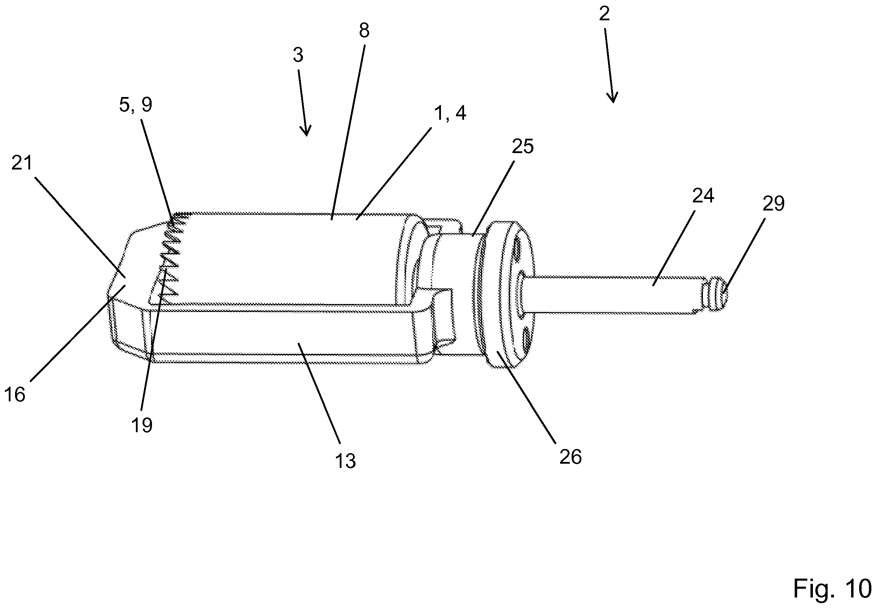

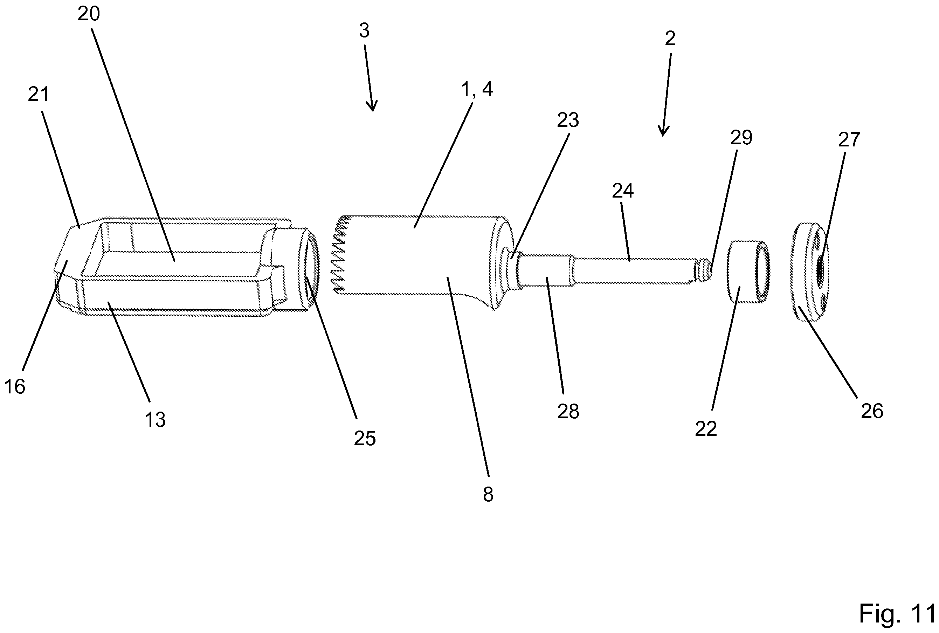

Die Figuren 10 und 11 zeigen ein weiteres Ausführungsbeispiel einer erfindungsgemäßen Schutzeinrichtung 13 nebst Werkzeug 1. In dem hier dargestellten Ausführungsbeispiel weist der distale Rand 5 des Werkzeugs 1 Zähne 19, insbesondere Schneidzähne bzw. Sägezähne auf, die als Wirkbereich 9 zur spanenden Bearbeitung von Knochen dienen. Dabei sind auch andere Ausgestaltungen des distalen Randes 5 denkbar, beispielsweise entsprechend den Figuren 1 bis 9.

Die Schutzeinrichtung 13 weist eine Rückwand 20 auf und ist an dem freien Ende 16 geschlossen, nämlich durch das Verdrängerelement 21. Das Verdrängerelement 21 dient dazu, die Knochenhaut während des Eingriffs wegzuschieben, so dass das oszillierende Werkzeug 1 möglichst einfach in den Knochen eindringen kann.

Ein weiterer Vorteil des Verdrängerelements 21 liegt darin, dass dieses den distalen Rand 5 des Werkzeugs 1 nochmals abschirmt, so dass das umliegende Gewebe bestmöglich vor Verletzungen geschützt ist. An dieser Stelle sei ausdrücklich darauf hingewiesen, dass das Verdrängerelement 21 nicht zwangsweise angeordnet sein muss, die Schutzeinrichtung 13 als solche den Arbeitsbereich 4 zumindest bereichsweise umgeben kann, um somit das umliegende Gewebe zu schützen.

In Figur 10 ist des Weiteren das Lager 22, beispielsweise ein Kugellager, Gleitlager etc., gezeigt, über welches die Schutzeinrichtung 13 mit dem proximalen Ende 2 des Werkzeugs 1, insbesondere dem Lagersitz 23 des Anschlussbereichs 24 bzw. Schafts, verbunden ist. Das Lager 22 kann auch als elastischer Ring realisiert sein, der sich bei einer Drehung zwischen Werkzeug 1 und Schutzeinrichtung 13 verformt. In der Schutzeinrichtung 13 kann eine Lageraufnahme 25 ausgebildet sein. Um eine sichere Verbindung der Schutzeinrichtung 13 mit dem Werkzeug 1, insbesondere in Axialrichtung, zu gewährleisten, ist ein Haltemittel 26 - bspw. eine Mutter - angeordnet, das mittels eines Innengewindes 27 mit dem Außengewinde 28 des Anschlussbereiches 24 verschraubbar ist.

Die Verbindung der Schutzeinrichtung 13 mit dem nicht dargestellten Handstück bzw. Winkelstück erfolgt somit über den Schaft bzw. Anschlussbereich 24 des Werkzeugs 1, welcher als Verbindungselement dient. Des Weiteren ist denkbar, dass die Schutzeinrichtung 13 alternativ oder zusätzlich über ein zusätzliches - nicht dargestelltes - Verbindungselement mit dem Handstück bzw. Winkelstück verbunden ist. Ein solches Verbindungselement hat den Effekt, dass die Schutzeinrichtung 13 drehfest an dem Winkelstück bzw. Handstück angeordnet ist, so dass ein "Mitdrehen" mit dem Werkzeug 1 vermieden wird.

Des Weiteren ist denkbar, dass ein Einlass 29 an dem Anschlussbereich 24 realisiert ist, der in Strömungsverbindung mit einem nicht dargestellte Auslass steht. Der Auslass kann insbesondere im Übergang zwischen proximalem Ende 2 und distalem Ende 3 des Werkzeugs 1 angeordnet sein kann. Somit kann das Werkzeug 1 während des Eingriffs gekühlt werden, um ein Überhitzen des Gewebes zu vermeiden.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Werkzeug
- 2: proximales Ende
- 3: distales Ende
- 4: Arbeitsbereich
- 5: distaler Rand
- 6: raue Oberfläche
- 7: innere Wandung
- 8: äußere Wandung
- 9: Wirkbereich
- 10: Winkel
- 11: Übergang
- 12: seitlicher Rand
- 13: Schutzeinrichtung
- 14: Drehachse
- 15: Führungselement
- 16: freies Ende
- 17: Steg
- 18: Vorsprung
- 19: Zähne
- 20: Rückwand
- 21: Verdrängerelement
- 22: Lager
- 23: Lagersitz
- 24: Anschlussbereich
- 25: Lageraufnahme
- 26: Haltemittel
- 27: Innengewinde
- 28: Außengewinde
- 29: Einlass

## Patentansprüche

1. Dentalchirurgisches Handgerät mit einem bewegbaren Werkzeug (1), wobei an einem distalen Ende (3) des Werkzeugs (1) ein Arbeitsbereich (4) ausgebildet ist und wobei zumindest ein distaler Rand (5) des Arbeitsbereichs (4) als Wirkbereich (9) dient,
**dadurch gekennzeichnet, dass** der Arbeitsbereich (4) zumindest bereichsweise als Hohlzylindersegment ausgebildet, so dass ein Block oder ein Zylindersegment aus dem Knochen heraustrennbar ist und dass eine Schutzeinrichtung (13) angeordnet ist, um das umliegende Gewebe vor Verletzungen durch das Werkzeug (1) zu schützen.

2. Dentalchirurgisches Handgerät nach Anspruch 1, **dadurch gekennzeichnet, dass** das Hohlzylindersegment des Arbeitsbereichs (4) eine Segmenthöhe von 2,5 mm bis 3,5 mm, insbesondere 3,1 mm bis 3,4 mm, vorzugsweise 3,2 mm aufweist.

3. Dentalchirurgisches Handgerät nach Anspruch 1 oder 2, **dadurch gekennzeichnet, dass** die Schutzeinrichtung (13) und/oder das Werkzeug (1) lösbar mit dem Handgerät, insbesondere mit einem Griff des Handgeräts, verbunden ist/sind.

4. Dentalchirurgisches Handgerät nach einem der Ansprüche 1 bis 3, **dadurch gekennzeichnet, dass** die Schutzeinrichtung (13) drehbar mit dem Werkzeug (2) gekoppelt ist.

5. Dentalchirurgisches Handgerät nach Anspruch 4, **dadurch gekennzeichnet, dass** die Schutzeinrichtung (13) über ein Lager (22), insbesondere ein Gleitlager bzw. Wälzlager bzw. Kugellager bzw. Rillenkugellager, oder über ein elastisches Element, insbesondere einen Ring, vorzugsweise aus Kunststoff bzw. Silikon, mit dem Werkzeug (2) gekoppelt ist.

6. Dentalchirurgisches Handgerät nach einem der Ansprüche 1 bis 5, **dadurch gekennzeichnet, dass** die Schutzeinrichtung (13) zumindest bereichsweise als Hohlzylindersegment ausgebildet ist.

7. Dentalchirurgisches Handgerät nach einem der Ansprüche 1 bis 6, **dadurch gekennzeichnet, dass** sich die Schutzeinrichtung (13) gegenüber der inneren Wandung (7) des Hohlzylindersegments des Werkzeugs (1) in Axialrichtung, insbesondere über den distalen Rand (5) hinweg, erstreckt.

8. Dentalchirurgisches Handgerät nach einem der Ansprüche 1 bis 7, **dadurch gekennzeichnet, dass** an dem distalen Rand (5) und/oder an der inneren Wandung (7) des Arbeitsbereichs (4) und/oder an der äußeren Wandung (8) des Arbeitsbereichs (4) eine raue Oberfläche (6) ausgebildet ist, beispielsweise eine Beschichtung mit Diamantkörnern oder Korundkörnern, die zumindest einen Teil des Wirkbereichs realisiert.

9. Dentalchirurgisches Handgerät nach einem der Ansprüche 1 bis 8, **dadurch gekennzeichnet, dass** an dem distalen Rand (5) Zähne (19) ausgebildet sind, die zumindest einen Teil des Wirkbereichs (9) realisieren.

10. Dentalchirurgisches Handgerät nach einem der Ansprüche 1 bis 9, **dadurch gekennzeichnet, dass** das Werkzeug (1) jeweils an einem Übergang (11) zwischen distalem Rand (5) und einem der seitlichen Ränder (12) des Hohlzylindersegments abgerundet ausgebildet ist.

11. Dentalchirurgisches Werkzeug mit einem proximalen Ende (2) zur Befestigung an einem chirurgischen Handgerät, wobei an einem distalen Ende (3) des Werkzeugs (1) ein Arbeitsbereich (4) ausgebildet ist und wobei zumindest ein distaler Rand (5) des Arbeitsbereichs (4) als Wirkbereich (9) dient,
**dadurch gekennzeichnet, dass** der Arbeitsbereich (4) zumindest bereichsweise als Hohlzylindersegment ausgebildet ist, so dass ein Block oder Zylindersegment aus dem Knochen heraustrennbar ist und dass eine Schutzeinrichtung (13) drehbar mit dem Werkzeug (1) gekoppelt ist, um umliegendes Gewebe vor Verletzungen durch das Werkzeug (1) zu schützen.

12. Werkzeug nach Anspruch 11, **dadurch gekennzeichnet, dass** die Schutzeinrichtung (13) über ein Lager (22), insbesondere ein Gleitlager bzw. Wälzlager bzw. Kugellager bzw. Rillenkugellager, oder über einen elastisches Element, insbesondere einen Ring, vorzugsweise aus Kunststoff bzw. Silikon, mit dem Werkzeug (1) gekoppelt ist.

## Claims

1. Dental surgical hand-held device having a movable tool (1), wherein an operating region (4) is constructed at a distal end (3) of the tool (1), and wherein at least one distal edge (5) of the operating region (4) acts as an active region (9), **characterised in that** the operating region (4) is constructed at least in regions as a hollow-cylindrical segment so that a block or a cylindrical segment can be separated from the bone and **in that** a protection device (13) is arranged in order to protect the surrounding tissue from injuries as a result of the tool (1).

2. Dental surgical hand-held device according to claim 1, **characterised in that** the hollow-cylindrical segment of the operating region (4) has a segment height of from 2.5 mm to 3.5 mm, in particular 3.1 mm to 3.4 mm, preferably 3.2 mm.

3. Dental surgical hand-held device according to claim 1 or claim 2, **characterised in that** the protection device (13) and/or the tool (1) is/are releasably connected to the hand-held device, in particular to a handle of the hand-held device.

4. Dental surgical hand-held device according to any one of claims 1 to 3, **characterised in that** the protection device (13) is rotatably coupled to the tool (2).

5. Dental surgical hand-held device according to claim 4, **characterised in that** the protection device (13) is coupled to the tool (2) by means of a bearing (22), in particular a plain bearing or roller bearing or ball bearing or deep-groove ball bearing, or by means of a resilient element, in particular a ring, preferably of plastics material or silicone.

6. Dental surgical hand-held device according to any one of claims 1 to 5, **characterised in that** the protection device (13) is constructed at least in regions as a hollow-cylindrical segment.

7. Dental surgical hand-held device according to any one of claims 1 to 6, **characterised in that** the protection device (13) extends with respect to the inner wall (7) of the hollow-cylindrical segment of the tool (1) in an axial direction, in particular over the distal edge (5).

8. Dental surgical hand-held device according to any one of claims 1 to 7, **characterised in that** at the distal edge (5) and/or on the inner wall (7) of the operating region (4) and/or on the outer wall (8) of the operating region (4) a rough surface (6) is constructed, for example, a coating with diamond grains or corundum grains, which forms at least a portion of the active region.

9. Dental surgical hand-held device according to any one of claims 1 to 8, **characterised in that** teeth (19) which form at least a portion of the active region (9) are constructed at the distal edge (5).

10. Dental surgical hand-held device according to any one of claims 1 to 9, **characterised in that** the tool (1) is constructed in each case in a rounded manner at a transition (11) between the distal edge (5) and one of the lateral edges (12) of the hollow-cylindrical segment.

11. Dental surgical tool having a proximal end (2) for securing to a surgical hand-held device, wherein an operating region (4) is constructed at a distal end (3) of the tool (1) and wherein at least one distal edge (5) of the operating region (4) acts as an active region (9),
**characterised in that** the operating region (4) is constructed at least in regions as a hollow-cylindrical segment so that a block or cylindrical segment can be separated from the bone and **in that** a protection device (13) is rotatably coupled to the tool (1) in order to protect surrounding tissue from injuries as a result of the tool (1).

12. Tool according to claim 11, **characterised in that** the protection device (13) is coupled to the tool (1) by means of a bearing (22), in particular a plain bearing or roller bearing or ball bearing or deep-groove ball bearing, or by means of a resilient element, in particular a ring, preferably of plastics material or silicone.

## Revendications

1. Appareil portatif de chirurgie dentaire avec un outil mobile (1) dans lequel, au niveau d'une extrémité distale (3) de l'outil (1), est réalisée une partie de travail (4) et dans lequel au moins un bord distal (5) de la partie de travail (4) sert de partie fonctionnement (9),
**caractérisé en ce que** la partie de travail (4) est conçue, au moins par endroits, comme un segment de cylindre creux, de façon à ce qu'un bloc ou un segment de cylindre puisse être extrait de l'os et **en ce qu'**un dispositif de protection (13) est disposé afin de protéger le tissu environnant de lésions dues à l'outil (1).

2. Appareil portatif de chirurgie dentaire selon la revendication 1, **caractérisé en ce que** le segment de cylindre creux de la partie de travail (4) présente une hauteur de segment de 2,5 mm à 3,5 mm, plus particulièrement de 3,1 mm à 3,4 mm, de préférence de 3,2 mm.

3. Appareil portatif de chirurgie dentaire selon la revendication 1 ou 2, **caractérisé en ce que** le dispositif de protection (13) et/ou l'outil (1) est/sont relié(s) de manière amovible avec l'appareil portatif, plus particulièrement avec une poignée de l'appareil portatif.

4. Appareil portatif de chirurgie dentaire selon l'une des revendications 1 à 3, **caractérisé en ce que** le dispositif de protection (13) est couplé de manière rotative avec l'outil (2).

5. Appareil portatif de chirurgie dentaire selon la revendication 4, **caractérisé en ce que** le dispositif de protection (13) est couplé avec l'outil (2) par l'intermédiaire d'un palier (22), plus particulièrement un palier lisse resp. un palier à rouleaux resp. un palier à billes resp. un palier rainuré à billes, ou par l'intermédiaire d'un élément élastique, plus particulièrement une bague, de préférence en matière plastique resp. silicone.

6. Appareil portatif de chirurgie dentaire selon l'une des revendications 1 à 5, **caractérisé en ce que** le dispositif de protection (13) est conçu, au moins par endroits, comme un segment de cylindre creux.

7. Appareil portatif de chirurgie dentaire selon l'une des revendications 1 à 6, **caractérisé en ce que** le dispositif de protection (13) s'étend, par rapport à la paroi interne (7) du segment de cylindre creux de l'outil (1), dans la direction axiale, plus particulièrement au-delà du bord distal (5).

8. Appareil portatif de chirurgie dentaire selon l'une des revendications 1 à 7, **caractérisé en ce que**, au niveau du bord distal (5) et/ou au niveau de la paroi interne (7) de la partie de travail (4) et/ou au niveau de la paroi externe (8) de la partie de travail (4), est réalisée une surface rugueuse (6), par exemple un revêtement avec des grains de diamants ou des grains de corindon, qui constitue au moins une partie de la partie fonctionnelle.

9. Appareil portatif de chirurgie dentaire selon l'une des revendications 1 à 8, **caractérisé en ce que**, au niveau du bord distal (5), sont réalisées des dents (19) qui constituent au moins une partie de la partie fonctionnelle (9).

10. Appareil portatif de chirurgie dentaire selon l'une des revendications 1 à 9, **caractérisé en ce que** l'outil (1) est conçu de manière arrondie respectivement au niveau d'une transition (11) entre un bord distal (5) et un des bords latéraux (12) du segment de cylindre creux.

11. Outil de chirurgie dentaire avec une extrémité proximale (2) pour la fixation à un appareil portatif de chirurgie dentaire, dans lequel, au niveau d'une extrémité distale (3) de l'outil (1), est réalisée une partie de travail (4) et dans lequel au moins un bord distal (5) de la partie de travail (4) sert de partie fonctionnelle (9),
**caractérisé en ce que** la partie de travail (4) est conçue, au moins par endroits, comme un segment de cylindre creux, de façon à ce qu'un bloc ou un segment de cylindre puisse être extrait de l'os et **en ce qu'**un dispositif de protection (13) est couplé de manière rotative avec l'outil (1) afin de protéger le tissu environnant de lésions dues à l'outil (1).

12. Outil selon la revendication 11, **caractérisé en ce que** le dispositif de protection (13) est couplé avec l'outil (1) par l'intermédiaire d'un palier (22), plus particulièrement un palier lisse resp. un palier à rouleaux resp. un palier à billes resp. un palier rainuré à billes, ou par l'intermédiaire d'un élément élastique, plus particulièrement une bague, de préférence en matière plastique resp. silicone.
